# EUROPEAN PATENT SPECIFICATION

(11) **EP 0 618 811 B1**
(45) Date of publication and mention of the grant of the patent: **05.04.2000**
(21) Application number: 93900778.7
(22) Date of filing: 02.12.1992
(51) Int. Cl.: A61K 38/54, C12N 9/50

(54) **PROTEOLYTIC MIXTURE CONTAINING ESCHARASE AND METHOD OF ISOLATING SAME**
ESCHARASE ENTHALTENE PROTEOLYTISCHE MISCHUNG UND METHODE SEINER ISOLIERUNG
MELANGE PROTEOLYTIQUE CONTENANT DE L'ESCHARASE ET SON PROCEDE D'ISOLEMENT

(30) Priority: 03.12.1991 US 801968
(43) Date of publication of application: 12.10.1994
(73) Proprietor: KLEIN, Gerold K. V., Brunswick, ME 04011 (US)
(72) Inventor: KLEIN, Gerold, K., V., Brunswick, ME 04011 (US); HOUCK, John, C., Seattle, WA 98105 (US)
(74) Representative: Weinhold, Peter, Dr.
(86) International application number: US9210395
(87) International publication number: WO9310811

(56) References cited:
- GB-A- 2 018 809
- GB-A- 2 093 867
- US-A- 3 817 834
- US-A- 4 329 430
- US-A- 4 602 913
- ROBERT K. SCOPES, "Protein Purification", published 1982, see pages 43-52.

## Description

### BACKGROUND OF THE INVENTION

Escharase is a hydrolytic enzyme material free of caseinolytic activity with an isoelectric point of about six. It has a molecular weight of about 45,000 daltons and is comprised of three subunits believed to be identical, each weighing about 15,000 daltons.

Escharase and mixtures containing escharase are useful for the debridement of devitalized tissue from a mammalian host. It is particularly useful for the removal of devitalized tissue from human burn victims.

The product, methods of isolation and methods of use are well known and are described for example by Houck, Chang and Klein in Int. J. Tiss. Reac. V(2) 125-134 (1983) and in United States Patents 4,197,291 issued April 8, 1980; 4,226,854 issued October 7, 1980 and 4,307,081 issued December 22, 1981.

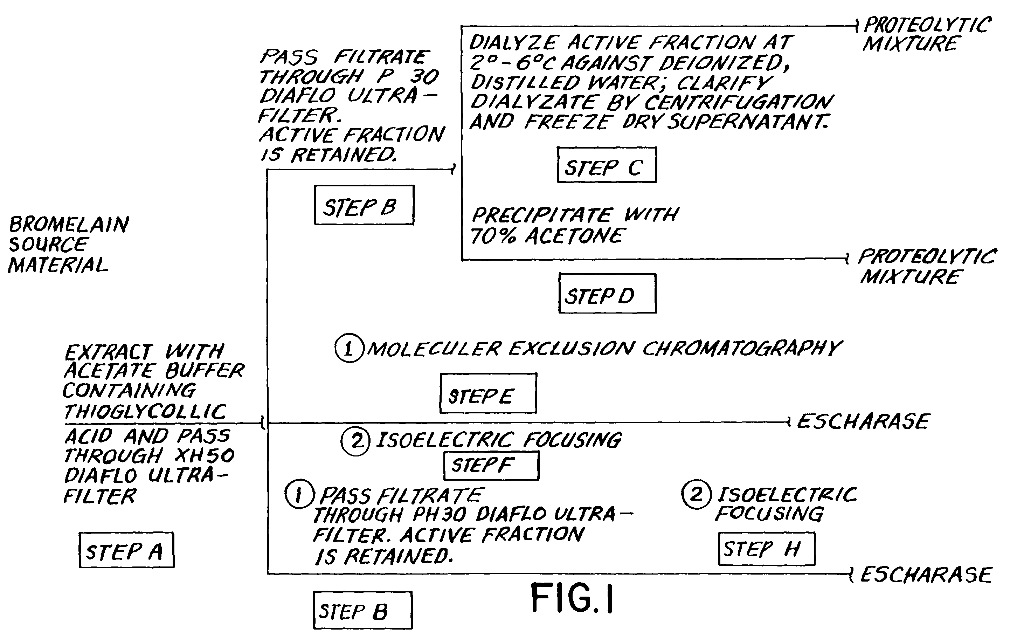

The Houck et al publication and the patents describe methods of isolating escharase itself and a proteolytic mixture containing escharase by the procedures generally illustrated in Fig. 1 hereof. The proteolytic mixture containing the escharase and, of course, the escharase itself are useful for the debridement of devitalized tissue from mammals.

As will be seen from the figure, the first step in the isolation process is to extract commercially available bromelain with an acetate buffer containing thioglycollic acid and then to filter. Specifically, the commercial bromelain is extracted (10 grams per 200 ml) in acetate buffer 0.1 M, pH 5.5 which has been made up to 1% in thioglycolic acid. The pH of this solution is approximately 4. The solution is expressed through XM 50 Amicon Diaflo ultrafilter (Amicon Corp., Boston, Mass.) and concentrated over PM 30 Diaflo filters. The active solution containing a mixture of proteolytic enzymes having molecular weights of from 30,000 to 50,000 dalton can be either dialyzed at 2° to 6°C against deionized, distilled water (200 volumes), clarified by centrifugation, and the clear supernatant freeze dried; or the active fraction can be precipitated with 70% acetone. Both procedures produce useful proteolytic mixtures.

As shown in Fig. 1, escharase can be isolated from the proteolytic enzyme mixture obtained after expression through XM 50 Amicon Diaflo ultrafilter as described above.

In one isolation procedure, the enzyme mixture is subjected to molecular exclusion chromatography as a phenylmercuric salt [prepared by combining the mixture with an aqueous 0.2 M citrate buffer saturated with the salt in accordance with the procedure of Ota et al in Biochem. 3:180 (1960)] on a column of Sephadex G 75. The elution of the escharase product from this column preceded the elution of pure stem bromelain, and therefore escharase must have a molecular weight in excess of bromelain, e.g. 32,000.

Sephadex G 75 is a polysaccharide gel available from Pharmacia of Upsala, Sweden. It is employed for molecular exclusion chromatography in accordance with procedures well known in the art.

The mixture obtained by exclusion chromatography is then fractionated by isoelectric focusing and subjected to polyacrylamide gel analytical eletrophoresis in 1% sodium diodecyl sulfate (SDS).

For isoelectric focusing, the mixture was mixed in a sucrose gradient with LKB Ampholine ampholytes initially from pH 3 to 10, and subsequently at pH 5-8. The active material was concentrated at a peak isoelectric point of pH 6.04, with a range from 5.85 to 6.12. This isoelectric point is markedly different from that described from bromelain (pH 4.7 and 9.9). See Vestberg Acta. Chem. Scand 20:820 (1966).

LKB Ampholine is available from the LKB Company of Sweden for isoelectric focusing. It is believed to be a mixture of small ampholytes.

The products isolated by isoelectric focusing have an extremely high order of escharase activity.

For further purification, the isoelectric focused, active material may be subjected to polyacrylamide gel electrophoresis at pH 9 in 1% (SDS) (Weber et al J. Viol. Chem 244:4406 (1969). Only one protein staining band can be visualized with a measured electrophoretic mobility which, when compared with standard proteins of known molecular weight, evidences a molecular weight of between 14,300 and 15,000 daltons. Since SDS is known to dissociate proteins into their various subunits if any, it is apparent that the escharase product of this invention comprises at least two, and most likely three subunits of substantially the same molecular weight.

The process of this invention makes available an improved proteolytic mixture containing much higher concentration of escharase. A particular advantage of the process of the invention is that it can be scaled up to use much larger quantities of the bromelain source. Moreover, it will consistently produce the same high yields. Additionally, it is more economical than the previous method because of the higher yields and the fact that process itself is less expensive.

As used in this description and claims, the term "bromelain" or "bromelain source" refers to any of a number of presently available bromelain preparations such as TMBC bromelain commercially available from Taiwan McKay. The bromelain is prepared from the stem of the pineapple plant. In the presently preferred procedure for obtaining bromelain for use in this invention, the juice from the stem is first adjusted to a pH of about 3 or 4 with phosphoric acid and, as described in the above identified patents and publication, sodium hydride or sodium sulfhydride is added to protect against sulfhydryl oxidation. The inert material is precipitated at about 30% acetone (addition of sufficient acetone so that the solution is 30% in acetone) and, after filtration, the clarified fluid is precipitated with 70% acetone. This precipitate is collected by centrifugation and either redissolved in water containing sodium hydride or sodium sulfhydride which has been acidified with phosphoric acid and reprecipitated, or dried in a vacuum oven directly. If the material is reprecipitated, 70% acetone is utilized. The dried material from either process is suitable as a starting material to obtain the products of this invention.

Dilute aqueous antioxidant solutions such as ascorbic acid refers to aqueous solutions containing from about 0.5 to 2% by weight ascorbic acid, preferably 0.75 to 1.25%, most preferably 1%.

Dilute aqueous ascorbic acid solutions are employed in the process of this invention to extract the bromelain source. The extract is clarified, for example by filtration and the proteolytic mixture containing the escharase is precipitated by the addition of a protein precipitant such as ammonium sulfate. Typically, to achieve efficient precipitation with ammonium sulfate, a sufficient amount of this reagent is added to form at least a 30% solution by weight, although the amount employed may be sufficient to form a saturated solution. Typically the solution will be from about 35% to 45% by weight in ammonium sulfate. Comparable amounts of other precipitants may be employed.

The escharase containing proteolytic mixture of this invention may be obtained from the bromelain source described above by extraction with an aqueous medium containing a non-volatile antioxidant. It is isolated from the extract and may be further purified. In the presently preferred procedure, the bromelain source is extracted with an aqueous solution of ascorbic acid at a pH of from about 3 to 4 and the desired product precipitated from the extract with a precipitant such as ammonium sulfate. The precipitated proteolytic mixture may be used as such, but it is preferred to further purify the mixture and concentrate its escharase content.

Although ascorbic acid is the preferred antioxidant because it is readily available, physiologically acceptable and extremely efficient, other antioxidants can be employed. These include, for example, dihydroquinone, butylated hydroxytoluene and dithiothreotol.

Similarly, other protein precipitants can be employed although, as with all such precipitations ammonium sulfate is by far the most preferred. These include both organic and inorganic precipitants including lower alkanols such as methanol and ethanol; acetone; polyethylene glycol; sulfates such as magnesium, potassium and sodium sulfate; and halides such as sodium and potassium chloride.

The selected antioxidant and precipitant should be reaction inert and should not denature the protein.

A typical procedure for the isolation of a proteolytic mixture of this invention is as follows:
1. 1 kilo of bromelain is blended (Waring Blender) for 60 seconds in 10 liters of 1% ascorbic acid (10 mg/ml) in distilled water at 4-10 C°.
2. This suspension is adjusted with 4N HCl to pH 3.5-3.9.
3. The suspension is stirred at 4-10 C° for 18 hours.
4. After separating the insoluble (20-25%) materials from the soluble fraction by filtration or centrifugation in the cold (4-10 C°) the soluble fraction is made up to 40% saturation with ammonium sulfate (2842gm/10L) and allowed to stand at 4-10 C° overnight.
5. After collecting the precipitate via centrifugation or filtration in the cold, the precipitate is dissolved in 10 liters of 0.3M acetic acid containing 0.1% ascorbic acid (1mg/ml) in the cold (pH 3.0).
6. This extract is then washed with 40 liters of distilled water over a 10,000 Dalton hollow fiber ultra-filter using an Amicon DC-30 A system with filter membrane cartilage type H10P1020, DF-40. A flow rate of 300 to 400 ml/min. at 19-23 psi at 10°C is maintained for about two hours and the final volume is reduced to about 4.5 to 5.0 liters.
7. The resulting solution is then lyophilized and weighed, usually about 250 gm yield.

The process of this invention provides a much improved escharase containing proteolytic mixture compared to the proteolytic mixture obtained by the process of the above identified patents and publication. The proteolytic mixture is obtained in much improved yields, for example about 25% yield compared to about 10% yield by the previous methods.

Of special significance, is the fact that the proteolytic mixture of the invention contains from about 1% to 1.5% escharase by weight based on the total weight of the mixture. The previously described methods provided proteolytic mixtures which, on average, contained much smaller amounts of escharase. Moreover, the mixture is stable over a longer period of time and appears to produce consistently better graftable beds for the acceptance of new skin than could be obtained from the previously described procedures.

The mixture may be characterized as containing from about 1% to 1.5% escharase together with proteolytic enzymes from bromelain extractable with dilute aqueous ascorbic acid.

If desired, escharase can be isolated from the proteolytic mixture of the invention by the following procedure:

### 1. G-75 Sephadex column chromatography

Dissolve 4 to 7 gm of proteolytic mixture in 50ml of 0.3M acetic acid containing 1 mg/ml of ascorbic acid. After centrifugation at 1200-1500 g the clear fluid is subjected to exclusion column chromatography (G-75) and the biologically active (by bioassay) fraction behind the void column and in front of the major peak rich in general protease activity (assayed on the denatured hemoglobin) is pooled, dialysed and lyophilised. This fraction elutes about where ovalbumin does i.e. 45,000 Daltons and contains about 15-20% of the applied proteolytic mixture.

### 2. Isoelectric focussing

The pooled, biologically active product from repeated G75 column runs is then subjected to isolectric focusing using a 0-40% sucrose gradient containing 1% LKB Ampholine in the LKB-8102 apparatus from pH 5 to 8 at 4°C as described in the above identified patents. During elution, the absorbence of the various fractions at 280 mu was determined and the various fractions pooled, dialysed and assayed. Only the fraction at pH 6.4 demonstrated the dissecting activity (i.e. separating denatured from native tissue). If the G-75 fraction is dissolved in 4M urea as a solvent, its isolectric point shifts from 6.4 to 6.8.

Repeated isoelectric focussing of this fraction gave a material absorbing u.v. at a peak of 280 mu which could dissect dead from live burned tissue to produce a graftable bed. This fraction was found to be electraphorectically homogenous at two different pH levels in acrylamide gel disc electrophoresis.

It is, of course, the same escharase isolated by the previously described procedures. It can dissect the plane between live and dead tissue but, has no general proteolytic activity against denatured hemoglobin, gelatin or casein. It also does not have hydrolytic activity against hyaluronic acid or dermatan sulfate, acid mucopolysaccharides of the skin.

Escharase is excluded from G-75 at a molecular weight of about 45,000 Daltons. After isoelectric focussing it appears to have a molecular weight on SDS-PAGE electrophoresis of about 15,000 and by Sephadex G-50 exclusion chromotagraphy of about 14,000 daltons. It therefor appears that Escharase is a complex of three identical subunits of about 15,000 Daltons which dissociate during isoelectric focussing.

The amino acid analysis of pure escharase is as follows:

| namomoles/nanomole of escharase (14,000 D) | |
|---|---|
| Alanine | 16 |
| arginine | 7 |
| aspartic acid | 12 |
| cystin/2 | 6.6 |
| glutamic acid | 10.6 |
| glycine | 16.8 |
| histidine | none |
| isoleucine | 6.4 |
| leucine | 6.4 |
| lysine | 5.6 |
| phenylalanine | 3.4 |
| proline | trace |
| serine | 14 |
| threonine | 7 |
| tyrosine | trace |
| vaLINE | 7.6 |
| methionine | none |
| Total | 115 nanomoles molecular weight of 13,800 |

Escharase is essentially devoid of methionine, histidine, proline and tyrosine. The lack of proline and tyrosine indicates escharase is a unique protein.

The precipitated escharase containing proteolytic mixture is recovered, for example by centrifugation and may be purified and concentrated by lyophilization, if desired. For this purification procedure, the precipitated proteolytic mixture is dissolved in acetic acid and lyophilized up to, for example, five times.

The proteolytic mixture of this invention and the escharase derived from it will be utilized for the removal of eschar utilizing the same procedures described in the above identified patents and publication.

Variations from the procedures described, reagents and instruments specifically described will be apparent to those skilled in the art. Other types of filters may be employed. Any of a variety of proteins may be employed as standards in electrophoresis. Sucrose is not the only gradient which can be employed in the isoelectric focusing procedures. The exact details of these procedures may vary from the equivalent procedures described above.

### EXAMPLE 1

A total of 250 grams of bromelain was homogenized for 60 seconds at 4°C with 2.5 liters of 1% aqueous ascorbic acid and a few drops of octanol was added to inhibit foaming. The pH was adjusted to 3.5 with 4N HCl. The mixture was then stirred gently for about 15 hours at 4°C and centrifuged at this same temperature for 30 minutes at 10,000 rpm. The supernate (2.525 liters) was collected and 742.4 grams of ammonium sulfate was added over a period of about 4 minutes with rapid stirring which was continued for another 10 minutes. The mixture was allowed to stand with gentle stirring at about 4°C for about fifteen hours. It was then separated by centrifugation for 30 minutes at 10,000 rpm at 4°C.

The precipitate was dissolved in 0.3 molar acetic acid containing 0.1% by weight ascorbic acid. The pH was adjusted to 3 with 4N HCl and the volume made up to 2500 ml by the addition of more aqueous acetic acid/ascorbic acid mixture and stirred at 4°C for 2 hours.

After standing at 4°C for about fifteen hours, the solution was concentrated and dialyzed on the CH2RS Amicon Concentrator with a 10,000 molecular weight cut-off spiral membrane. Ten to 12 liters of cold 0.3 molar acetic acid was added at pH 3 over a period of a few hours. The final concentrate was brought to one half volume (1.25 liters) and the ammonium ion content was found to be negligible with EMI dipsticks (Nessler's reaction). The concentrate was centrifuged at 4°C for 30 minutes at 10,000 rpm. The supernate was stored for about 15 hours at -70°C and lyophilized at about 25°C and at 7mm Hg. The yield of product, based on the weight of the original starting material was 29%.

### EXAMPLE 2

Standard burn wounds were created on an anesthetized piglet by applying 360° heat to 4.4 cm areas of skin for 45 seconds. The blistered keratin was removed by brushing with dry gauze. The burns were kept moist with Normal saline soaks until they were treated.

The burn area was isolated from the unburned area by a frame which was secured in place. The burn was soaked with Normal saline and 200 mg of a powder prepared substantially as described in Example 1 were sprinkled evenly over the surface of the burn. The powder was dissolved by dropwise addition of Normal Saline and protected by coating with a sterile gel. The gel was then covered with another plastic net and more gel was added. The application was then sealed with Saran wrap to maintain Anaerobic conditions.

The area was warmed with heating lamp to maintain a temperature of about 102°F.

After four hours, the covering was removed and the eschar wiped with Normal Saline soaked gauze. There resulted an excellent digestion and dissection of eschar. Blotting removed most of the proteolyzed eschar leaving only a few patches of remnant eschar with much basal circulation visible. After 20 wipes with dry gauze, almost all of the eschar was removed with increased amounts of punctuate bleeding points visible. All of the eschar was removed by an additional 20 wipes with Normal Saline soaked gauze leaving a clean bed except for a few strands of remaining eschar. After ten minutes of warm Normal Saline washing, the wound bed appeared clean and visibly graftable. A few strains of basal eschar were still visible at this point, yet not enough to hinder a graft take.

The graftability of the bed was established by standard histological examination which confirmed less than 0.1 mm residual central eschar and excellent debridement.

The experiment was repeated with the proteolytic mixture obtained by the process of the above identified U.S. Patents. It was observed that although the bed was still graftable, the amount of central eschar remaining was from 0.1 to 0.2 mm.

## Claims

1. A method for preparing a proteolytic mixture containing escharase, said method comprising:
(a) extracting a bromelain preparation with a dilute aqueous antioxidant solution at a pH of from 3 to 4; and
(b) precipitating the desired proteolytic mixture from the acidic extract formed in step (a) by adding a protein precipitant thereto.

2. Method according to claim 1 wherein the antioxidant is ascorbic acid.

3. Method according to any one of claims 1 or 2 wherein the protein precipitant is ammonium sulfate.

4. A proteolytic mixture containing escharase obtainable by:
(a) extracting a bromelain preparation with a dilute aqueous antioxidant solution at a pH of from 3 to 4; and
(b) precipitating the desired proteolytic mixture from the acidic extract formed in step (a) by adding a protein precipitant thereto.

5. Proteolytic mixture according to claim 4 wherein the antioxidant of step (a) is ascorbic acid.

6. Proteolytic mixture according to any one of claims 4 or 5 wherein the protein precipitant of step (b) is ammonium sulfate.

7. Proteolytic mixture according to any one of claims 4 to 6 which contains from 1% to 1.5% escharase.

## Patentansprüche

1. Ein Verfahren zur Herstellung einer proteolytischen Mischung, welche Escharase enthält, wobei das Verfahren die folgenden Schritte umfaßt:
(a) Extrahieren einer Bromelain-Präparation mit einer verdünnten wäßrigen Lösung eines Antioxidationsmittels bei einem pH von 3 bis 4; und
(b) Fällen der gewünschten proteolytischen Mischung aus dem sauren Extrakt, welcher in Schritt (a) gebildet wurde, durch Hinzugeben eines Proteinfällungsmittels.

2. Verfahren gemäß Anspruch 1, wobei das Antioxidationsmittel Ascorbinsäure ist.

3. Verfahren gemäß einem der Ansprüche 1 oder 2, wobei das Proteinfällungsmittel Ammoniumsulfat ist.

4. Eine proteolytische Mischung, welche Escharase enthält, die erhältlich ist durch das:
(a) Extrahieren einer Bromelain-Präparation mit einer verdünnten wäßrigen Lösung eines Antioxidationsmittels bei einem pH von 3 bis 4; und
(b) Fällen der gewünschten proteolytischen Mischung aus dem sauren Extrakt, welcher in Schritt (a) gebildet wurde, durch Hinzugeben eines Proteinfällungsmittels.

5. Proteolytische Mischung gemäß Anspruch 4, wobei das Antioxidationsmittel aus Schritt (a) Ascorbinsäure ist.

6. Proteolytische Mischung gemäß einem der Ansprüche 4 oder 5, wobei das Proteinfällungsmittel aus Schritt (b) Ammoniumsulfat ist.

7. Proteolytische Mischung gemäß einem der Ansprüche 4 bis 6, welche von 1% bis 1,5% Escharase enthält.

## Revendications

1. Procédé pour préparer un mélange protéolytique contenant de l'escharase, ledit procédé comprenant :
(a) l'extraction d'une préparation de bromélaïne avec une solution aqueuse diluée d'anti-oxydant à un pH de 3 à 4 ; et
(b) la précipitation du mélange protéolytique désiré à partir de l'extrait acide formé dans l'étape (a) par addition d'un agent de précipitation de protéine à celui-ci.

2. Procédé suivant la revendication 1, dans lequel l'anti-oxydant est l'acide ascorbique.

3. Procédé suivant l'une quelconque des revendications 1 ou 2, dans lequel l'agent de précipitation de protéine est le sulfate d'ammonium.

4. Mélange protéolytique contenant de l'escharase pouvant être obtenu par :
(a) extraction d'une préparation de bromélaïne avec une solution aqueuse diluée d'anti-oxydant à un pH de 3 à 4 ; et
(b) précipitation du mélange protéolytique désiré à partir de l'extrait acide formé dans l'étape (a) par addition d'un agent de précipitation de protéine à celui-ci.

5. Mélange protéolytique suivant la revendication 4, dans lequel l'anti-oxydant de l'étape (a) est l'acide ascorbique.

6. Mélange protéolytique suivant l'une quelconque des revendications 4 ou 5 dans lequel l'agent de précipitation de protéine de l'étape (b) est le sulfate d'ammonium.

7. Mélange protéolytique suivant l'une quelconque des revendications 4 à 6 qui contient de 1% à 1,5% d'escharase.
